Europäisches Patentamt

European Patent Office

Office européen des brevets

(11) Veröffentlichungsnummer: **0 312 793**
**A1**

(12) **EUROPÄISCHE PATENTANMELDUNG**

(21) Anmeldenummer: 88115614.5

(22) Anmeldetag: 22.09.88

(51) Int. Cl.⁴: **A62D 3/00 , C12R 1/645**

(30) Priorität: 22.09.87 DE 3731816

(43) Veröffentlichungstag der Anmeldung:
26.04.89 Patentblatt 89/17

(84) Benannte Vertragsstaaten:
AT BE CH ES FR GB GR IT LI LU NL SE

(71) Anmelder: **G.A. Pfleiderer**
**Unternehmensverwaltung GmbH & Co. KG**
**Ingolstädter Strasse 51 Postfach 1480**
**D-8430 Neumarkt/Opf.(DE)**

(72) Erfinder: **Hüttermann, Aloys, Prof. Dr.**
**Büsgenweg 2**
**D-3400 Göttingen(DE)**
Erfinder: **Trojanowski, Jerzy, Prof. Dr.**
**Büsgenweg 2**
**D-3400 Göttingen(DE)**
Erfinder: **Loske, Dagmar, Dipl.-Biol.**
**Rosenwinkel 7a**
**D-3400 Göttingen(DE)**

(74) Vertreter: **Harders, Gerhard, Dr.**
**Stettiner Strasse 2**
**D-6367 Karben 6(DE)**

(54) **Verfahren zum Abbau schwer abbaubarer Aromaten in kontaminierten Böden bzw. Deponiestoffen mit Mikroorganismen.**

㊗ Dadurch gekennzeichnet, daß bodenfremde ligninabbauende Weißfäulepilze mit vorwiegend hemicellulosehaltigen Abfallstoffen, die einer Druck- und Temperaturbehandlung in Gegenwart von Wasserdampf unterzegen Worden sind, vermischt und das erhaltene Gemisch in die kontaminierten Böden bzw.Deponiestoffe eingebracht wird.

EP 0 312 793 A1

# Verfahren zum Abbau schwer abbaubarer Aromaten in kontaminierten Böden bzw. Deponiestoffen mit Mikroorganismen

Die vorliegende Erfindung betrifft ein Verfahren zum Abbau schwer abbaubarer Aromaten in kontaminierten Böden bzw, Deponiestoffen mit Mikroorganismen.

Bei der Handhabung von aromatischen Verbindungen kommt es vor, beispielsweise durch Unachtsamkeit, Beschädigung von Lager- und Transportgefäßen, Unfälle und dergleichen, daß aromatische Verbindungen in die Umwelt und insbesonin den Boden gelangen. Auch bei aufgelassenen Industriestandorten stellt sich häufig das Problem, daß insbesondere bei ehemaligen Chemiebetrieben, aber auch bei Kokereien etc. die Böden in hohem Maße mit organischen Verbindungen, insbesondere mit aromatischen Verbindungen belastet sind. Dies führt dann meist dazu, daß solche Standorte bei der weiteren Umwidmung den Strukturwandel unmöglich machen, da sie stark gesundheitsgefährdend sind, von den Problemen für die Grundwasserbelastung ganz abgesehen.

Bisher gibt es für die Sanierung solcher Böden keine zufreidenstellende Technologie. Kleinere Menge kontaminierter Böden lassen sich in Verbrennungsanlagen entgiften, bei größeren Menge besteht die einzig mögliche Sanierung in der Ausbaggerung und dem Abtransport in Sondermülldeponien. Das eigentliche Entsorgungsproblem wird dadurch nur zeitlich und räumlich verschoben.

Es ist bekannt, daß bestimmte im Boden Vorkommende Bakterien Aromaten abzubauen vermögen. So ist in Experientia 39, S. 1231-1236, 1983 beschrieben, daß Arthrobacter ATCC 33790 in Schlämmen, also in Flüssig-Systemen, die Fähigkeit hat, Pentachlorphenol abzubauen.

Solche Systeme, die bisher nur in der Flüssigphase erprobt wurden, sind in ihrer Anwendung sehr beschränkt. So wird beispielsweise der bakterielle Abbau von p-Nitrobenzoat bereits durch die Anwesenheit von nicht nitriertem Benzoat in Schlämmen gehemmt. In diesem Zusammenhang ist darauf zu verweisen, daß durch Bakterien bewirkte Umsetzungen durch intrazelluläre Enzyme in der Bakterienzelle katalysiert werden. Der Abbau von Aromaten, insbesondere von Chloraromaten, erfolgt im allgemeinen nicht bis zur völligen Mineralisation der Aromaten, in die Huminstoffe des Bodens werden sie partiell eingebaut. Die Mineralisierung wird durch Stickstoffmangel gehemmt.

In der EU-OS 0 192 237 ist ein Verfahren zum Abbau von Bodenverunreinigungen beschrieben, in dem die Bodenverunreinigungen mit Pilzenzymen, z. B. von Weißfäulepilzen, die eine Lignin abbauende Oxygenase und Wasserstoffperoxid enthalten, unter aeroben Bedingungen umgesetzt werden. Dabei können die Verunreinigungen mit Lignin, Cellulose, Altpapier, Holzspänen, Sägemehl oder Humus vermischt werden. Ein Vermischen mit hemicellulosehaltigen Materialien ist in dieser Druckschrift nicht beschrieben.

Aufgabe der vorliegenden Erfindung ist es, ein Entsorgungssystem für schwer abbaubare Aromaten in kontaminierten Böden bzw. Deponiestoffen zu schaffen, mit dem die Kontaminationen direkt, d.h. ohne Zwischenschaltung einer flüssigen Phase, entsorgt werden können. Dabei soll das Entsorgungskonzept einen möglichst breiten Anwendungsbereich umfassen, ohne durch andere Aromaten gehemmt zu werden.

Gelöst wird diese Aufgabe dadurch, daß bodenfremde ligninabbauende Weißfäulepilze mit vorwiegend hemicellulosehaltigen Abfallstoffen, die einer Druck-und Temperaturbehnadlung unterzogen worden sind, vermischt werden und das erhaltene Gemisch in die kontaminierten Böden bzw. Deponiestoffe eingebracht wird.

Überraschenderweise entfalten die bodenfremden liginiabbauenden Weißfäulepilze in festen Substraten, wie kontaminierten Böden bzw. Deponiestoffen, ohne Zwischenschaltung einer flüssigen Phase in Gegenwart von teilweise aufgeschlossenen Hemicellulosen eine starke Aromaten-abbauende Wirkung mit breitem Wirkungsspektrum. Die Mineralisierung wird, anders als beim üblichen Ligninabbau, nicht durch Stickstoffmangel gehemmt, auch die Gegenwart anderer Aromaten hat keine Hemmwirkung. Die Mineralisierung führt nahezu vollständig zu organischen Substanzen, Chloraromaten werden vollständig zu Chlor bzw. Chlorid abgebaut.

Weißfäulepilze sind bodenfremd, d. h., sie wachsen im allgemeinen nicht im Boden, sondern auf Holz. Im Boden entfalten sie ohne Zugabe von externem Substrat keinerlei Aromaten-abbauende Wirkung. Nur wenn sie ganz besondere Mycelformen ausbilden, wie etwa Rhizomorphen bei Armillaria, können sie überhaupt in den Boden eindringen und die Strecke bis zum nächsten Baum überbrücken. Bei Zugabe von Lignocellulosen ist die Abbaurate gering.

Es wurde nun überraschenderweise gefunden, daß bei Zugabe von teilweise aufgeschlossenem hemicellulosehaltigen Material in kontaminierten Böden bzw. Deponiestoffen durch Weißfäulepilze ein guter Aromaten-Abbau bewirkt wird. Da hemicellulosehaltige Materialien allein ohne Anwesenheit von Weißfäulepilzen keinen Abbau der Aromaten bewirken, handelt es sich um einen synergistischen Effekt der Weißfäulepilze zusammen mit dem hemicellulosehaltigen Material, der zum Abbau der Aromaten führt.

Als hemicellulosehaltige Abfallstoffe werden beispeilsweise Stroh, Spelzen oder Bagasse eingesetzt.

Als bodenfremde liginiabbauende Weißfäulepilze werden bevorzugt folgende Gattungen bzw. -Arten eingesetzt: Armillaria, Clitocybe, Ganoderma, Lenzites, Panus, Pholiota, Pleurotus, Polyporus, Polystictus, Poria, Stereum, Trametes, Ischnoderma, Hypholoma, Ustulina, Heterobasidion, Phlebia, Hischioporus, Stereum, Coriolus.

Die Abfallstoffe werden einer kombinierten Druck-und Temperaturbehandlung in Gegenwart von Wasserdampf unterzogen, beispielsweise in einem Autoklaven. Die Druckbehandlung und Temperaturbehandlung kann z. B. bei Temperaturen von 100 bis 140°C erfolgen, wobei sich in einem abgeschlossenen System ein Druck von etwa 1 bis 3,6 bar einstellt, wenn Wasserdampf zugegen ist. Bevorzugt werden Temperaturen von etwa 105 bis 130°C verwendet, mit einem Druck von etwa 1,2 bis 2,7 bar.

Hohe Drucke und Temperaturen sollen nach Möglichkeit vermieden werden, da hierbei Glucose entstehen kann, die den Aromatenabbau hemmt. Deshalb ist ein Niederdruck-Aufschluß bevorzugt, während Hemicellulosen sonst bevorzugt bei drastischen Bedingungen aufgeschlossen wurden, beispielsweise mit starken Säuren bei hohen Temperaturen und Drucken, z. B. bei 187°C und 12 bar über einen Zeitraum von mehreren Stunden. Beim erfindungsgemäßen Verfahren genügen hingegen Aufschlußzeiten von wenigen Minuten, z. B. von 2 bis 100 Minuten, bevorzugt von 5 bis 60 Minuten, jeweils in Abhängigkeit von den verwendeten Temperaturen und Drucken.

Die Behandlung führt zu einer Teilautolyse und Quellung der hemicellulosehaltigen Materialien. Durch diese Behandlung wird die Hemicellulose für die eingesetzten Weißfäulepilze in eine verfügbare Form überführt.

Die Weißfäulepilze können entweder in Form wässriger Susupensionen eingesetzt werden, oder sie können auf Getreidekörner aufgegeben werden, wodurch ein leicht handhabbares Granulat entsteht./

Die zu entsorgenden kontaminierten festen Substrate werden, beispielsweise bei kontaminierten Böden, ausgebaggert und mit den vorbehandelten hemicellulosehaltigen Abfallstoffen, die vorher mit den lignin abbauenden Pilzen inkubiert worden sind, beispielsweise durch Aufsprühen der Suspension oder durch Vermischen mit Granulat. Die so erhaltene Mischung wird in üblicher Weise aufgeschichtet und kompostiert, entwerder offen an der Luft oder unter einer Abdeckung, beispielsweise einer Plastikfolie. Falls der durch die Bodendurchmischung und das Abfallmaterial eingebrachte Luftsauerstoff nicht für einen oxidativen Abbau der organischen Stoffe ausreicht, muß der Komposthaufen hin und wieder belüftet werden. Nach Beendigung des Vorgangs kann der Boden wieder zurückgeführt und ein weiteres Segment der Gesamtfläche kompostiert werden. Infolge des erhöhten Gehalts an organischer Substanz sind die so kompostierten Böden für alle üblichen Bodennutzungen einschließlich Garten- und Gemüsebau geeignet, sofern nicht eine zusätzliche Schwermetallbelastung vorliegt.

Die wässrigen Suspensionen der ligninabbauenden Pilze weisen einen Feststoffgehalt von mindestens etwa 0,01 bis etwa 10 Gew.-% auf, bevorzugt von etwa 0,05 bis 5 Gew.-% und insbesondere von etwa 0,1 bis 0,5 Gew.-%. Die wässrigen Suspensionen werden in einer Menge von mindestens etwa 1 Gew.-% auf die Abfallstoffe aufgebracht. Dabei sind Mengen von etwa 3 bis 20 Gew.-% bevorzugt und von etwa 5 bis 10 Gew.-% besonders bevorzugt.

Zur besseren Dosierung können beispielsweise wässrige Suspensionen in den genannten Mengen auf Getreidekörner, z. B. von Weizen, Roggen, Gerste oder Hafer aufgebracht werden und vorteilhaft bis zu 14 Tagen inkubiert werden.

Auf 100 Gewichtsteile des kontaminierten Bodens bzw. des Deponiestoffes werden mindestens etwa 1 Gewichtsteil des mit Suspension bzw, mit Granulat versehenen Abfallstoffes eingesetzt, bevorzugt etwa 1 bis 20 Gewichtsteile und besonders bevorzugt etwa 5 bis 10 Gewichtsteile.

Die vorbehandelten hemicellulosehaltigen Abfallstoffe stellen ein für den schnellen Abbau von Aromaten unbedingt notwendiges Cosubstrat bereit, das von den Pilzen sehr leicht genutzt werden kann. Zusammen mit dem Belüftungsfaktor werden somit optimale Bedingungen für den Abbau von ansonsten nur schwer abbaubaren Substanzen geschaffen.

Eine zusätzliche Belüftung, auch in periodischer Form, kann den Wirkungsgrad des Abbaus verbessern. Ebenso kann es vorteilhaft sein, insbesondere bei tiefen Außentemperaturen, dem Kompostierungsvorgang zusätzlich Wärme zuzuführen.

Das erfindungsgemäße Verfahren ermöglicht des Abbau aller aromatischen Verbindungen, insbesondere von hochkondensierten aromatischen Systemen mit hoher Giftigkeit, wie z.B. Anthracen- und Benzpyren-Derivaten.

Die Erfindung wird nachfolgend anhand der Beispiele näher erläutert, wobei alle in den Beispielen enthaltenen Merkmale als erfindungswesentlich angesehen werden.

3

Beispiel 1

Anzucht der Weißfäulepilze

Von Agarplattenkulturen der Versuchsorganismen wurden kleine Mengen Mycel (5x5 mm) steril abgenommen und in 30 ml Flüssigmedium BSM in 500 ml Erlenmeyerkolben überführt. Das Flüssigmedium BSM ist wie folgt zusammengesetzt:

5 g Glucose
o,5 g Hefeextrakt
0,5 g Asparagin
1 g $KH_2PO_4$
0,5 g $MgSO_4.7H_2O$
0,5 g KCl
0,01 g $FeSO_4.7H_2O$ 0,008g $Mn(CH_3COO)_2.4H_2O$
0,002g $ZnNO_3$
0,05 g $Ca(NO_3)_2.4H_2O$
0,003g $CuSO_4.5H_2O$
ad 1000ml dest. Wasser

Von diesen Stammkulturen ausgehend wurden für die jeweiligen Versuche neue Kulturen angesetzt, nachdem das Mycel ausreichend oberflächendeckend gewachsen war, was im allgemeinen nach 4 bis 8 Tagen der Fall war.

Für Versuche mit festen Bodensubstraten (Ackerboden) wirde das Mycel von den Stammkulturen in 5 ml Wasser zerkleinert und auf zerkleinertes Stroh überimpft, das 20 min bei 120° in einem abgeschloseenen System mit Wasserdampf behandelt worden ist.

Beispiel 2

Die Kulturen von Beispiel 1 wurden nach Erreichen eines guten oberflächendeckenden Wachstums zerkleinert und auf sterilisierte Weizenkörner überimpft.

Das so erhaltene Granulat kann wie die Suspension nach Beispiel 1 mit vorbehandeltem Stroh eingesetzt werden.

Beispiel 3

Abbauversuche mit verschiedenen Weißfäulepilzen

Die inkubierten Substrate nach den Besipielen 1 und 2 wurden etwa 5 bis 12 Tage in Ruhe belassen, bis das Mycel ausreichend gewachsen war. Dann wurde 1 g des Substrats mit 10 g Ackerboden (Lehm-Löß) vermischt und mit 0,5 bzw. 0,1 mg $^{14}$C-markiertem Anthracen versetzt. Das Anthracen wurde als wässrige Suspension zugesetzt.

Dabei wurden die in der Tabelle 1 genannten Abbauraten erhalten (bestimmt durch die Entwicklung von [14]$CO_2$).

Wasserlösliche Abbauprodukte, die aus hydrophoben Aromaten durch die Pilzkulturen produziert wurden, konnten durch eine Filtration durch Millipore-Filter erfaßt und mit einem $^{14}$C-Szintillationsfilter bestimmt werden.

Tab. 1

| Abbau von schwer abbaubaren Aromaten durch lignolytische Pilze in festen Substraten mit Zusatz von Stroh | | | |
|---|---|---|---|
| | Aromat: $^{14}$C-(Seitenring)-Anthracen | | |
| | Konzentration im Boden Gew.% | Freisetzung: | |
| | | $^{14}$CO$_2$ | $^{14}$C-Produk wasserlöslic |
| | | % | % |
| Trametes versicolor | 0,001 | 32,4 | 42,3 |
| " " | 0,005 | 12,1 | 31,0 |
| Polyporus pinsitus | 0,001 | 22,8 | 34,5 |
| " " | 0,005 | 12,0 | 22,0 |
| Pleurotus florida Stamm PP | 0,001 | 28,0 | 42,1 |
| " " " " | 0,005 | 11,0 | 30,2 |
| Bjerkandera adusta | 0,001 | 13,8 | 28,8 |
| " " | 0,005 | 10,7 | 17,2 |
| Hypholoma fasciculare | 0,001 | 18,2 | 15,4 |
| Pleurotus florida Stamm F6 | 0,001 | 8,6 | 19,1 |
| " " " " | 0,005 | 4,5 | 11,9 |
| Pleurotus florida Stamm P2 | 0,001 | 5,4 | 17,4 |
| " " " " | 0,005 | 3,5 | 15,0 |
| Pleurotus ostreatus | 0,005 | 4,6 | 9,7 |
| Pycnoporus sanguineus | 0,005 | 4,1 | 39,3 |
| Kontroll I: mit Pilzmycel ohne Stroh | 0,005 | 0.1-0,3 | n.b. |
| Kontrolle II: mit Stroh unsteril | 0,005 | 0,3-0,5 | n.b. |
| Kultivationszeit: 57 Tage | | | |

Beispiel 4

Es wurde wie in Beispiel 2 verfahren, wobei als Weißfäulepilze Pleurotus florida, Trametes versicolor und Polyporus pinsitus eingesetzt wurden.

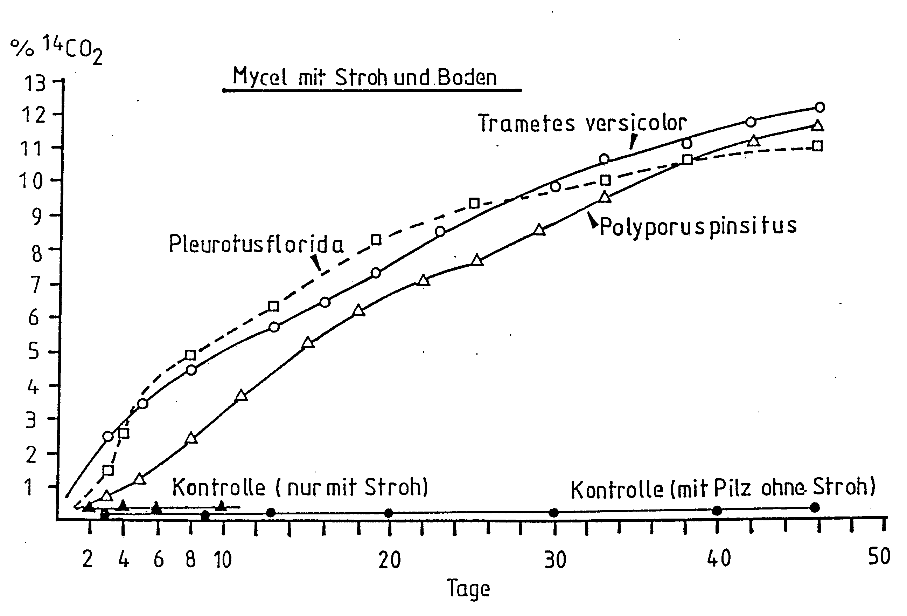

Die Abbauversuche wurden wie in Beispiel 3 durchgeführt. Dabei wurden die in der Abbildung 1 dargestellten Abbauraten erhalten. Der Abbau der Aromaten wurde durch die Bestimmung der $^{14}$CO$_2$-Freisetzung erfaßt.

Bei den Versuchen mit Ackerboden wurden parallel zwei Kontrollproben eingesetzt:

Kontrolle 1: Ackerboden mit Pilzmycel ohne Strohzusatz
Kontrolle 2: Ackerboden mit Strohzusatz ohne Pilzmycel, Boden und Stroh nicht vorbehandelt.

Beispiel 5

Vergleich der Abbauraten von Anthracen im Boden bei Behandlung mit vorbehandelter Cellulose, vorbehandelter Lignocellulose (Sägemehl), vorbehandeltem Hemicellulosen-Substrat (Stroh) und unbehandeltem Stro

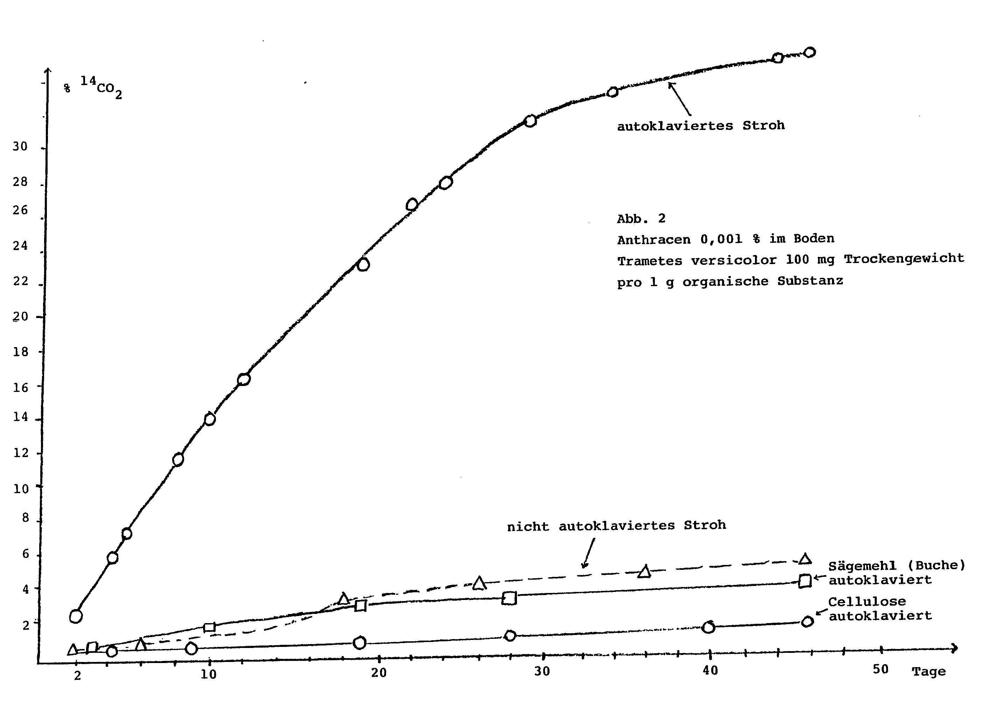

Es wurde wie in Beispiel 2 verfahren, wobei als Weißfäulepilz Trametes versicolor eingesetzt wurde.
Die Abbauversuche wurden wie in Beispiel 3 durchgeführt. Dabei wurden die in der Abbildung 2 dargestellten Abbauraten erhalten. Der Abbau der Aromaten wurde durch die Bestimmung der $^{14}$CO$_2$-Freisetzung erfaßt.

Beispiel 6

Abbauversuche mit sehr schwer kontaminiertem Erdreich

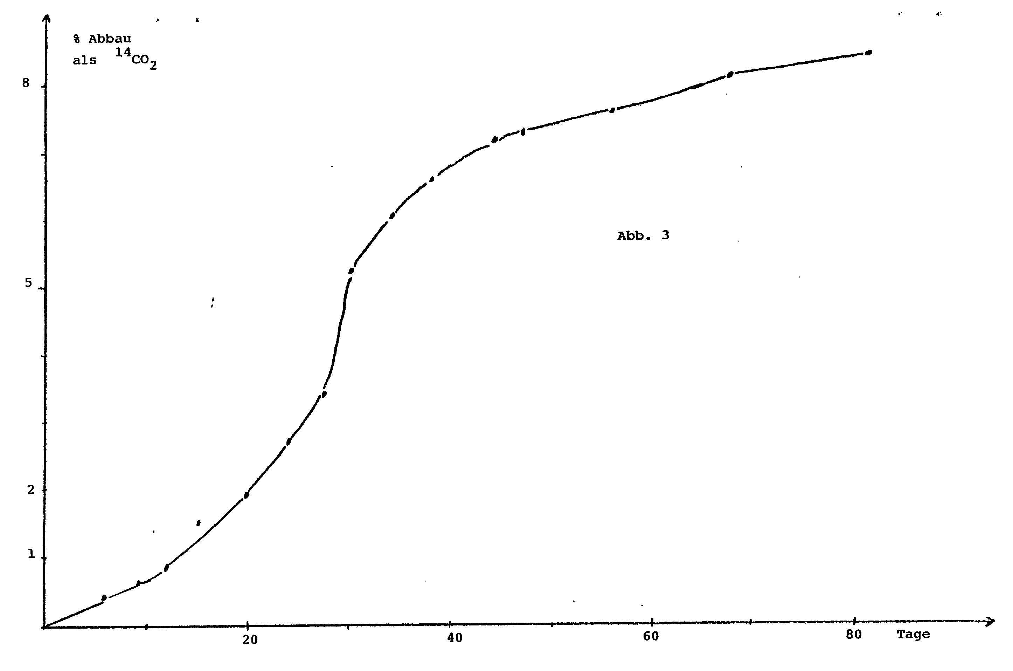

Das nach Beispiel 1 bzw. 2 inkubierte Stroh wurde etwa 5 bis 12 Tage in Ruhe belassen, bis das Mycel von Trametes versicolor ausreichend gewachsen war. Dann wurden 20 g des Strohs mit 180 g eines Bodens aus einer ehemaligen Teerfabrik vermischt. Dieser Boden war außerordentlich stark mit aromatischen Verbindungen belastet, allein die Belastung mit den sieben wichtigsten und toxischsten (carcinogenen) polyaromatischen Kohlenwasserstoffen betrug 1,6 g pro kg. Zusätzlich wurde $^{14}$C-markiertes Anthracen zugemischt. Dabei wurden die in der Figure 3 dargestellten $CO_2$-Entwicklungen gemessen, wobei nach 80 Tagen 8 % des Anthracens zu $CO_2$ abgebaut waren. Nach 80 Tagen wurden die aromatischen Inhaltsstoffe mit Äthylacetat aus dem Boden extrahiert und die Konzentrationen der oben genannten polyaromatischen Kohlenwasserstoffe (PAK) durch Hockdruck-Flüssigkeits-Chromatographie mit Fluoreszenzdetektoren gemessen. Dabei wurden die in der Tabelle 2 aufgeführten Werte erhalten. Der Gesamtgehalt der PAK wurde um 25 Gew.-% abgebaut. Es zeigte sich, daß die tatsächlich gemessenen Abbauraten ganz deutlich über der entsprechenden [14]$CO_2$-Freisetzung liegen.

Tabelle 2

| Abbauversuche mit Trametes versicolor, Polyaromatengehalt des Bodens in % | | | | |
|---|---|---|---|---|
| PAK | Menge mg | kg %-Gehalt PAK bei Behandlung mit | | |
| | | Boden | Stroh ohne Pilze | Stroh mit Pilzen |
| Anthracen | 180 | 100 | 106 | 54 |
| Fluoranthen | 467 | 100 | 98 | 59 |
| Benzo(b)fluoranthen | 136 | 100 | 107 | 63 |
| Benzo(k)fluoranthen | 139 | 100 | 99 | 64 |
| Benzo(a)pyren | 232 | 100 | 100 | 67 |
| Indeno(ghi)pyren | 165 | 100 | 105 | 71 |
| Benzoperylen | 290 | 100 | 89 | 79 |

**Ansprüche**

1. Verfahren zum Abbau schwer abbaubarer Aromaten in kontaminierten Böden bzw. Deponiestoffen mit Mikroorganismen, **dadurch gekennzeichnet, daß** bodenfremde ligninabbauende Weißfäulepilze mit vorwiegend hemicellulosehaltigen Abfallstoffen, die einer Druck- und Temperaturbehandlung in Gegenwart von Wasserdampf unterzogen worden sind, vermischt und das erhaltene Gemisch in die kontaminierten Böden bzw. Deponiestoffe eingebracht wird.

2. Verfahren nach Anspruch 1, dadurch gekennzeichnet, daß als vorwiegend hemicellulosehaltige Abfallstoffe Stroh, Spelzen oder Bagasse eingesetzt werden.

3. Verfahren nach Anspruch 1, dadurch gekennzeichnet, daß wässrige Suspensionen der ligninabbauenden Pilze mit einem Feststoffgehalt von mindestens etwa 0,01 bis etwa 10 Gew.-% eingesetzt werden.

4. Verfahren nach Anspruch 1, dadurch gekennzeichnet, daß die wässrigen Suspensionen der ligninabbauenden Pilze in einer Menge von mindestens etwa 1 Gew.-% auf die Abfallstoffe aufgebracht werden.

5. Verfahren nach Anspruch 1, dadurch gekennzeichnet, daß auf 100 Gew.-Teile des kontaminierten Bodens bzw. der Deponiestoffe mindestens 1 Gew.-Teil des mit Suspension versehenen Abfallstoffs eingesetzt werden.

6. Verfahren nach Anspruch 1, dadurch gekennzecihnet, daß die Mischung aus kontaminiertem Boden bzw. Deponiestoff und mit Suspension versehenem Abfallstoff während des Abbaus der Aromaten belüftet wird.

7. Verfahren nach den Ansprüchen 1 bis 6, dadurch gekennzeichnet, daß die Suspension in Form eines auf Getreidekörener aufgesprühten Granulats verwendet wird.

8. Verfahren Nach Anspruch 1, dadurch gekennzeichnet, daß die Druck- und Temperaturbehandlung in einem abgeschlossenen System bei Temperaturen von etwa 100 bis 140° C erfolgt.

9. Verfahren nach Anspruch1, dadurch gekennzeichnet, daß die Druck- und Temperaturbehandlung über einen Zeitraum von etwa 5 bis 100 min erfolgt.

EP 0 312 793 A1

Abb. 2
Anthracen 0,001 % im Boden
Trametes versicolor 100 mg Trockengewicht
pro 1 g organische Substanz

% Abbau als $^{14}CO_2$

Abb. 3

20    40    60    80    Tage

EP 0 312 793 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 554 075 (H. CHANG et al.) <br> * Ansprüche * <br> --- | 1-9 | A 62 D 3/00 <br> C 12 R 1/645 |
| D,A | EP-A-0 192 237 (BOARD OF TRUSTEES OF MICHIGAN STATE UNIVERSITY) <br> * Seite 7, Zeilen 10-24; Seite 22, Zeile 36 - Seite 23, Zeile 19; Ansprüche * <br> --- | 1-9 | |
| A | CHEMICAL ABSTRACTS, Band 102, Nr. 19, 13. Mai 1985, Seite 493, Zusammenfassung Nr. 165220j, Columbus, Ohio, US; I. ANNELE et al.: "Cultivation of wood-rotting fungi on agricultural lignocellulosic materials for the production of crude protein", & AGRIC. WASTES 1985, 12(2), 81-97 <br> * Zusammenfassung * <br> --- | 1-9 | |
| D,A | EXPERIENTIA, Band 39, Nr. 11, 15. November 1983, Seiten 1231-1236, Basel, CH; R.K. FINN: "Use of specialized microbial strains in the treatment of industrial waste and in soil decontamination" <br> * Seite 1232, rechte Spalte, Zeilen 15-34 * <br> --- | 1-9 | RECHERCHIERTE SACHGEBIETE (Int. Cl.4) <br><br> A 62 D <br> C 12 R |
| A | WO-A-8 601 843 (K.E. ERIKSSON et al.) <br> * Ansprüche * <br> ----- | 1-9 | |

Der vorliegende Recherchenbericht wurde für alle Patentansprüche erstellt

| Recherchenort | Abschlußdatum der Recherche | Prüfer |
|---|---|---|
| DEN HAAG | 14-12-1988 | FLETCHER A.S. |